# EUROPEAN PATENT APPLICATION

(11) **EP 4 238 576 A1**
(43) Date of publication of application: **06.09.2023**
(21) Application number: 21922507.5
(22) Date of filing: 30.11.2021
(51) Int. Cl.: A61K 38/39, A61K 47/54, A61K 47/61, A61P 19/02

(54) **COMPOSITE CONTAINING NON-DENATURING TYPE II COLLAGEN AND CHITOSAN OLIGOSACCHARIDE, AND PREPARATION METHOD THEREFOR**

(30) Priority: 28.01.2021 CN 202110114882
(71) Applicant: Ci, Qu, Beijing 100013 (CN)
(72) Inventor: GUISHI, Zhang, Handan, Hebei 057405 (CN); CI, Qu, Beijing 100013 (CN)
(74) Representative: Gulde & Partner
(86) International application number: PCT/CN2021/134442
(87) International publication number: WO 2022/160917

(57) **Abstract**

Disclosed in the present application are a complex containing undenatured type II collagen and chitosan oligosaccharide, and a preparation method therefor. The preparation method comprises the following steps: (1) activation: mixing a certain amount of powder or particulate matter containing undenatured type II collagen with an activating agent and storing the mixture at 0°C to 80°C for 1 min to 300 min; (2) sieving: sieving the mixture obtained in step (1) to remove unreacted activating agent; (3) coupling: adding chitosan oligosaccharide to the mixture obtained in step (2) in which the unreacted activating agent is removed, and dynamically mixing them at 0°C to 80°C for 1 min to 300 min; and (4) drying: drying the mixture obtained in step (3) and controlling the water content to be 8% or less, the resulting product being a complex containing undenatured type II collagen and chitosan oligosaccharide. This preparation method is simple to implement, with fewer operation steps and relatively high efficiency.

## Description

### TECHNICAL FIELD

The present application belongs to the protein complex preparation technique in biotechnology, and specifically to a complex containing undenatured type II collagen and chitosan oligosaccharide and a preparation method therefor.

### BACKGROUND OF THE INVENTION

Collagen is a natural biopolymer with a triple helical structure expressed by animal cells. As a major component of the extracellular matrix, it is found in almost all tissues, constituting approximately one-third of the total protein in mammals, and is widely distributed in the skin, Achilles tendon, cartilage, and other connective tissues of animals. Collagen plays a supporting and connecting role within tissues and affects cellular differentiation, proliferation, migration, and adhesion. It also has significant influences on tissue morphology, mechanical strength, and metabolism within tissues. Currently, twenty-eight distinct types of collagens have been identified in mammals. Distinct types of collagens exhibit distinct distribution patterns, existence forms, synthesis, and metabolic pathways in animals.

Type II collagen is predominantly found in tissues such as articular cartilage and bone and is primarily secreted by chondrocytes in animals. Type II collagen is a fibril-forming collagen that intertwines into a sturdy meshwork structure within the body to maintain the structural integrity of cartilage and to provide tension and load-bearing capacity to the cartilage tissue. Type II collagen possesses specific molecular recognition signals, such that it can promote the adhesion, proliferation, and differentiation of chondrocytes, and is more conducive than type I collagen to the chondrocyte phenotype formation and structural maintenance. Abnormalities in the expression or metabolism of type II collagen, and denaturation of its native structure can lead to a variety of diseases.

Rheumatoid arthritis is a disease associated with an autoimmune response against type II collagen, which is a chronic autoimmune disease primarily characterized by joint synovitis. The key to its pathological mechanism lies in the recurrent inflammation of the joints. The pathological features of rheumatoid arthritis mainly include synovial lining cell proliferation, abundant infiltration of inflammatory cells in the interstitium, neovascularization and formation, as well as destruction of cartilage and bone tissue. Rheumatoid arthritis can cause a variety of diseases in the human body that are rich in type II collagen, which is an immune disease.

Oral administration of type II collagen has been shown to have a significant effect on the prevention and treatment of rheumatoid arthritis by inducing immune tolerance. Type II collagen immune determinants can bind to antigens in the body to form complexes, which are then presented on the surface of antigen-presenting cells through the secretory pathway for recognition by the surface receptors of the receptor-specific immune-related cells to form a composite. The specific immune-related cells are activated, proliferate, and secrete various immune factors to initiate an immune response, resulting in specific immunity of local intestine-related lymphoid tissues and thus suppressing the systemic immune response, representing a form of peripheral immune tolerance.

Traditional type II collagen, when orally administered, can undergo degradation in the stomach, resulting in a decrease of targeted release rate in the intestines. Additionally, the distribution of amino acids on the surface of native type II collagen is predominantly negatively charged, which hinders its phagocytosis by cells on the surface of the intestines. As a result, the effectiveness of traditional type II collagen in oral form is often limited. Enhancing the targeted release ratio of undenatured type II collagen in the intestines can increase the phagocytosis rate by intestinal cells through appropriate pathways, and thus improve the therapeutic efficacy.

### SUMMARY OF THE INVENTION

In response to the limitation of the therapeutic effect on arthritis caused by the low release ratio and low phagocytosis rate of traditional undenatured type II collagen in use, the present application provides a complex containing undenatured type II collagen and chitosan oligosaccharide and a preparation method therefor.

According to an aspect of the present application, provided is a method for preparing a complex containing undenatured type II collagen and chitosan oligosaccharide, which includes the following steps:
(1) activation: mixing a certain amount of powder or particulate matter containing undenatured type II collagen with an activating agent and storing the mixture at 0°C to 80°C for 1 min to 300 min;
(2) sieving: sieving the mixture obtained in step (1) to remove unreacted activating agent;
(3) coupling: adding chitosan oligosaccharide to the mixture obtained in step (2) in which the unreacted activating agent is removed, and dynamically mixing them at 0°C to 80°C for 1 min to 300 min;
(4) drying: drying the mixture obtained in step (3) and controlling the water content to be 8% or less, such that the resulting product is a complex containing undenatured type II collagen and chitosan oligosaccharide.

Preferably, the activating agent added in the step (1) is daidzein, soy isoflavone, anthocyanidin, anthocyanin, tea polyphenol, dihydromyricetin, or plant extracts containing the above substances.

Preferably, the mass ratio of the activating agent to the undenatured type II collagen is 100:1 to 1:10000.

Preferably, the chitosan oligosaccharide added in the step (3) has a degree of polymerization of 2 to 10.

Preferably, the chitosan oligosaccharide added in the step (3) is a mixture of chitosan oligosaccharides with varying degrees of polymerization, with an average degree of polymerization ranging from 2 to 10.

Preferably, the mass ratio of the chitosan oligosaccharide added in the step (3) to the undenatured type II collagen is 100:1 to 1:200.

Preferably, in the step (2), the mixture obtained in the step (1) is sieved using a screen.

According to another aspect of the present application, provided is a complex containing undenatured type II collagen and chitosan oligosaccharide prepared by the preparation method described in the above embodiment.

Preferably, the undenatured type II collagen and chitosan oligosaccharide in the complex are present in a chemically bonded form.

Preferably, the ratio of the undenatured type II collagen to chitosan oligosaccharide is in a range of 1:200 to 100:1.

The method for preparing a complex containing undenatured type II collagen and chitosan oligosaccharide in embodiments of the present application has advantages of simple implementation process, fewer operation steps and high efficiency. An embodiment of the present application involves immobilizing chitosan oligosaccharide on the surface of undenatured type II collagen using an activating agent and increasing the density of positive charges on the surface of the undenatured type II collagen. Therefore, after the complex containing undenatured type II collagen and chitosan oligosaccharide in the embodiment of the present invention is orally administered, the undenatured type II collagen contained in the complex is less susceptible to degradation by proteases in the human body, thereby enhancing the targeted release ratio of the undenatured type II collagen in the intestines. Additionally, the inclusion of undenatured type II collagen in the complex of the embodiment of the present application facilitates the phagocytosis of collagen by the cells on the surface of the intestines, thereby improving the phagocytosis rate of the undenatured type II collagen by intestinal cells, and further enhancing the therapeutic effect.

### DETAILED DESCRIPTION

The present application will be described in detail below by specific embodiments. It should be noted that the embodiments are only examples of the complex containing undenatured type II collagen and chitosan oligosaccharide and the preparation method therefor in the present application, which cannot represent the entirety of the patent of this invention and should not be construed as the limitation to the scope of protection for this invention. Some adjustments or improvements can also be made under the premise of not departing from the essential concept of this invention, all of which fall within the scope of protection for this invention.

According to general concept of the present application, provided is a method for preparing a complex containing undenatured type II collagen and chitosan oligosaccharide, which includes the following steps:
(1) activation: mixing (e.g., evenly) a certain amount of powder or particulate matter containing undenatured type II collagen (e.g., powder or particulate matter obtained from pretreatment of animal cartilages) with an activating agent and storing the mixture at 0°C to 80°C (e.g., 35°C to 40°C, or 50°C to 55°C) for 1 min to 300 min (e.g., 90 min or 200 min);
(2) sieving: sieving the mixture obtained in step (1) to remove unreacted activating agent;
(3) coupling: adding chitosan oligosaccharide to the mixture obtained in step (2) in which the unreacted activating agent is removed, and dynamically mixing them at 0°C to 80°C (e.g., 35°C to 40°C, or 50°C to 55°C) for 1 min to 300 min (e.g., 90 min or 120 min);
(4) drying: drying the mixture obtained in step (3) and controlling the water content to be 8% or less (e.g., 7% or less, or 6% or less), the resulting product being a complex containing undenatured type II collagen and chitosan oligosaccharide.

In the preparation method according to embodiments of the present application, chitosan oligosaccharide is immobilized on the surface of undenatured type II collagen by using an activating agent, making the undenatured type II collagen less susceptible to degradation by proteases in the human body after oral administration, thereby enhancing the targeted release ratio of the undenatured type II collagen in the intestines. Additionally, the addition of the activating agent increases the density of positive charges on the surface of the undenatured type II collagen, so the inclusion of undenatured type II collagen in the complex facilitates the phagocytosis of collagen by the cells on the surface of the intestines, thereby improving the phagocytosis rate of the undenatured type II collagen by intestinal cells. The complex obtained by the preparation method of the present application enhances the therapeutic effect of undenatured type II collagen.

In the embodiments, the activating agent added in the step (1) is flavone, anthocyanidin, polyphenols or plant extracts containing the above substances.

In the embodiments, the mass ratio of the activating agent to the undenatured type II collagen powder or particulate matter is 100:1 to 1:10000, e.g., 1:10.

In the embodiments, the chitosan oligosaccharide added in the step (3) has a degree of polymerization of 2 to 10, e.g., 3 or 4.

In an example, the chitosan oligosaccharide added in the step (3) is a mixture of chitosan oligosaccharides with varying degrees of polymerization, with an average degree of polymerization ranging from 2 to 10, e.g., 3 or 4.

In an example, the mass ratio of the chitosan oligosaccharide added in the step (3) to the undenatured type II collagen powder or particulate matter is 100:1 to 1:200, e.g., 2.5:1 or 1:5.

In the embodiments, in the step (2), the mixture obtained in the step (1) is sieved using a screen. For example, the mixture obtained in the step (1) is sieved using a screen of 200 to 500 meshes.

According to another concept of the present application, also provided is a complex containing undenatured type II collagen and chitosan oligosaccharide prepared by the preparation method in any one of the above embodiments.

In the embodiments, the undenatured type II collagen and chitosan oligosaccharide in the complex are present in a chemically bonded form.

In the embodiments, the ratio of the undenatured type II collagen to chitosan oligosaccharide is in a range of 1:200 to 100:1.

Only a part of the embodiments of the present application are shown below for exemplary illustration; other feasible embodiments of the present application will be understood by those skilled in the art and will not be repeated here.

### Embodiment 1

Particulate matter containing 20% of undenatured type II collagen obtained from pretreatment of animal cartilage was used as the raw material, of which the water content was about 20%, and the activating agent used was soy isoflavone.
(1) Activation: 200 g of chicken cartilage powder with an average particle size of 300 mesh was mechanically mixed with 4 g of soy isoflavone evenly, and the resulting mixture was stored at 35°C to 40°C for 200 min.
(2) Sieving: the mixture obtained in step (1) was sieved using a 500-mesh screen and the retentate was collected.
(3) Coupling: 100 g of chitosan oligosaccharide with an average degree of polymerization of 4 was added into the retentate obtained in step (2) and mixed by shaking at 35°C to 40°C for 200 min.
(4) Drying: the mixture obtained in step (3) was dried in vacuum at low temperature until its water content reached 7% or less.

The mixture contained a complex of undenatured type II collagen and chitosan oligosaccharide, in which the chitosan oligosaccharide was covalently coupled to the surface of the undenatured type II collagen. The test results showed that the ratio of undenatured type II collagen to chitosan oligosaccharide in the complex was 22.3:1.

### Embodiment 2

Lyophilized powder of undenatured type II collagen extracted from chicken cartilage was used as the raw material, of which the water content was about 10%, and the activating agent used was blueberry extract containing 20% of dihydromyricetin, of which the water content was about 6%.
(1) Activation: 100 g of lyophilized powder of undenatured type II collagen was mechanically mixed with 10 g of blueberry extract evenly, and the resulting mixture was stored at 50°C to 55°C for 90 min.
(2) Sieving: the mixture obtained in step (1) was sieved using a 200-mesh screen and the lyophilized powder of undenatured type II collagen was collected.
(3) Coupling: 20 g of chitosan oligosaccharide with an average degree of polymerization of 3 was added into the lyophilized powder obtained in step (2) and mixed by shaking at 50°C to 55°C for 120 min.
(4) Drying: the mixture obtained in step (3) was dried in vacuum at low temperature until its water content reached 6% or less.

The mixture contained a complex of undenatured type II collagen and chitosan oligosaccharide. The test results showed that the ratio of undenatured type II collagen to chitosan oligosaccharide in the complex was 20.6:1.

## Claims

1. A method for preparing a complex containing undenatured type II collagen and chitosan oligosaccharide, comprising the following steps:
(1) activation: mixing a certain amount of powder or particulate matter containing undenatured type II collagen with an activating agent and storing the mixture at 0°C to 80°C for 1 min to 300 min;
(2) sieving: sieving the mixture obtained in step (1) to remove unreacted activating agent;
(3) coupling: adding chitosan oligosaccharide to the mixture obtained in step (2) in which the unreacted activating agent is removed, and dynamically mixing them at 0°C to 80°C for 1 min to 300 min;
(4) drying: drying the mixture obtained in step (3) and controlling the water content to be 8% or less, the resulting product being a complex containing undenatured type II collagen and chitosan oligosaccharide.

2. The preparation method according to claim 1, wherein the activating agent added in the step (1) is flavone, anthocyanidin, polyphenols or plant extracts containing the above substances.

3. The preparation method according to claim 2, wherein the mass ratio of the activating agent to the undenatured type II collagen is 100:1 to 1:10000.

4. The preparation method according to any one of claims 1 to 3, wherein the chitosan oligosaccharide added in the step (3) has a degree of polymerization of 2 to 10.

5. The preparation method according to any one of claims 1 to 3, wherein the chitosan oligosaccharide added in the step (3) is a mixture of chitosan oligosaccharides with varying degrees of polymerization, with an average degree of polymerization ranging from 2 to 10.

6. The preparation method according to any one of claims 1 to 3, wherein the mass ratio of the chitosan oligosaccharide added in the step (3) to the undenatured type II collagen is 100:1 to 1:200.

7. The preparation method according to any one of claims 1 to 3, wherein, in the step (2), the mixture obtained in the step (1) is sieved using a screen.

8. A complex containing undenatured type II collagen and chitosan oligosaccharide prepared by the preparation method according to any one of claims 1 to 7.

9. The complex containing undenatured type II collagen and chitosan oligosaccharide according to claim 8, wherein the undenatured type II collagen and chitosan oligosaccharide in the complex are present in a chemically bonded form.

10. The complex containing undenatured type II collagen and chitosan oligosaccharide according to claim 9, wherein the ratio of the undenatured type II collagen to chitosan oligosaccharide is in a range of 1:200 to 100:1.
